# EUROPEAN PATENT APPLICATION

(11) **EP 1 905 449 A2**
(43) Date of publication of application: **02.04.2008**
(21) Application number: 07121768.1
(22) Date of filing: 04.04.2000
(51) Int. Cl.: A61K 39/00, A61K 39/39, A61P 31/04, A61P 31/12, A61P 35/00, A61P 37/08, A61P 25/28

(54) **Vaccines**

(30) Priority: 19.04.1999 GB 9908885; 29.04.1999 US 301829
(62) Divisional of application: 04076239.5
(71) Applicant: GlaxoSmithKline Biologicals S.A., 1330 Rixensart (BE)
(72) Inventor: Friede, Martin, 1330 Rixensart (BE); Garcon, Nathalie, 1330 Rixensart (BE); Hermand, Philippe, 1330 Rixensart (BE)
(74) Representative: Hambleton, Bernadette Angelina

(57) **Abstract**

The present invention relates to adjuvant compositions which are suitable to be used in vaccines. In particular, the adjuvant compositions of the present invention comprises a saponin and an immunostimulatory oligonucleotide, optionally with a carrier. Also provided by the present invention are vaccines comprising the adjuvants of the present invention and an antigen. Further provided are methods of manufacture of the adjuvants and vaccines of the present invention and their use as medicaments. Methods of treating an individual susceptible to or suffering from a disease by the administration of the vaccines of the present invention are also provided

## Description

The present invention relates to novel adjuvant compositions for use in vaccines. In particular, the adjuvant compositions of the present invention comprise a combination of saponin and an immunostimulatory oligonucleotide, said combination optionally further comprising a carrier. Also provided by the present invention are vaccines comprising the adjuvant compositions of the present invention and at least one antigen. Further provided are methods of manufacture of the adjuvant compositions and vaccines of the present invention and their use as medicaments. Additionally, the present invention provides methods of treating an individual susceptible to or suffering from a disease by the parenteral or mucosal administration of the vaccines of the present invention.

Immunostimulatory oligonucleotides containing unmethylated CpG dinucleotides ("CpG") and are known in the art as being adjuvants when administered by both systemic and mucosal routes (WO 96/02555, EP 468520, Davis et al., J.Immunol, 1998, 160(2):870-876; McCluskie and Davis, J.Immunol., 1998, 161(9):4463-6). CpG is an abbreviation for cytosine-guanosine dinucleotide motifs present in DNA. Historically, it was observed that the DNA fraction of BCG could exert an anti-tumour effect. In further studies, synthetic oligonucleotides derived from BCG gene sequences were shown to be capable of inducing immunostimulatory effects (both in vitro and in vivo). The authors of these studies concluded that certain palindromic sequences, including a central CG motif, carried this activity. The central role of the CG motif in immunostimulation was later elucidated in a publication by Krieg, Nature 374, p546 1995. Detailed analysis has shown that the CG motif has to be in a certain sequence context, and that such sequences are common in bacterial DNA but are rare in vertebrate DNA. The immunostimulatory sequence is often: Purine, Purine, C, G, pyrimidine, pyrimidine; wherein the dinucleotide CG motif is not methylated, but other unmethylated CpG sequences are known to be immunostimulatory and may be used in the present invention.

In certain combinations of the six nucleotides a palindromic sequence is present. Several of these motifs, either as repeats of one motif or a combination of different motifs, can be present in the same oligonucleotide. The presence of one or more of these immunostimulatory sequence containing oligonucleotides can activate various immune subsets, including natural killer cells (which produce interferon γ and have cytolytic activity) and macrophages (Wooldrige et al Vol 89 (no. 8), 1977). Although other unmethylated CpG containing sequences not having this consensus sequence have now been shown to be immunomodulatory.

CpG when formulated into vaccines, is generally administered in free solution together with free antigen (WO 96/02555; McCluskie and Davis, *supra*) or covalently conjugated to an antigen (PCT Publication No. WO 98/16247), or formulated with a carrier such as aluminium hydroxide ((Hepatitis surface antigen) Davis *et al. supra* ; Brazolot-Millan et al., Proc.Natl.Acad.Sci., USA, 1998, 95(26), 15553-8).

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, *supra*). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1.

Particulate structures, termed Immune Stimulating Complexes (ISCOMS), comprising fractions of Quil A are haemolytic and have been used in the manufacture of vaccines (Morein, B., EP 0 109 942 B1). These structures have been reported to have adjuvant activity (EP 0 109 942 B1; WO 96/11711).

The haemolytic saponins QS21 and QS17 (HPLC purified fractions of Quil A) have been described as potent systemic adjuvants, and the method of their production is disclosed in US Patent No.5,057,540 and EP 0 362 279 B1. Also described in these references is the use of QS7 (a non-haemolytic fraction of Quil-A) which acts as a potent adjuvant for systemic vaccines. Use of QS21 is further described in Kensil et al. (1991. J. Immunology vol 146, 431-437). Combinations of QS21 and polysorbate or cyclodextrin are also known (WO 99/10008). Particulate adjuvant systems comprising fractions of QuilA, such as QS21 and QS7 are described in WO 96/33739 and WO 96/11711.

Other saponins which have been used in systemic vaccination studies include those derived from other plant species such as Gypsophila and Saponaria (Bomford et al., Vaccine, 10(9):572-577, 1992).

Saponins are also known to have been used in mucosally applied vaccine studies, which have met with variable success in the induction of immune responses. Quil-A saponin has previously been shown to have no effect on the induction of an immune response when antigen is administered intranasally (Gizurarson et al. 1994. Vaccine Research 3, 23-29). Whilst, other authors have used this adjuvant with success (Maharaj et al., Can.J.Microbiol, 1986, 32(5):414-20; Chavali and Campbell, Immunobiology, 174(3):347-59). ISCOMs comprising Quil A saponin have been used in intragastric and intranasal vaccine formulations and exhibited adjuvant activity (McI Mowat et al., 1991, Immunology, 72, 317-322; McI Mowat and Donachie, Immunology Today, 12, 383-385).

QS21, the non-toxic fraction of Quil A, has also been described as an oral or intranasal adjuvant (Sumino et al., J. Virol., 1998, 72(6):4931-9; WO 98/56415).

The use of other saponins in intranasal vaccination studies has been described. For example, *Chenopodium quinoa* saponins has been used in both intranasal and intragastric vaccines (Estrada et al., Comp. Immunol. Microbiol. Infect. Dis., 1998, 21(3):225-36).

The present invention relates to the surprising finding that immunostimulatory oligonucleotides (CpG) and saponin combinations are extremely potent adjuvants. Accordingly, there is provided an adjuvant composition comprising a combination of saponin and an immunostimulatory oligonucleotide. Preferably, the adjuvants of the present invention may further comprise a carrier. In a preferred form of the present invention the saponin and oligonucleotides in the adjuvant and vaccine compositions act synergistically in the induction of antigen specific antibody and are potent in the induction of immune responses conventionally associated with the Th1-type immune system. Accordingly, the adjuvant combinations are not only suitable for immunoprophylaxis of diseases, but also surprisingly for immunotherapy of diseases such as persistant viral, bacterial or parasitic infections, and also chronic disorders such as cancer.

The preferred oligonucleotides for use in adjuvants or vaccines of the present invention preferably contain two or more dinucleotide CpG motifs separated by at least three, more preferably at least six or more nucleotides. The oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention including oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages.
OLIGO 1 (SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006)
OLIGO 5 (SEQ ID NO:5): TCC ATG ACG TTC CTG ATG CT (CpG 1668)

Alternative CpG oligonucleotides may comprise the preferred sequences above in that they have inconsequential deletions or additions thereto.
The CpG oligonucleotides utilised in the present invention may be synthesized by any method known in the art (eg EP 468520). Conveniently, such oligonucleotides may be synthesized utilising an automated synthesizer.

The oligonucleotides utilised in the present invention are typically deoxynucleotides. In a preferred embodiment the internucleotide bond in the oligonucleotide is phosphorodithioate, or more preferably phosphorothioate bond, although phosphodiesters are within the scope of the present invention. Oligonucleotide comprising different internucleotide linkages are contemplated, e.g. mixed phosphorothioate phophodiesters. Other internucleotide bonds which stabilise the oligonucleotide may be used.

The saponins which may be used in the adjuvant combinations of the present invention include those derived from the bark of *Quillaja Saponaria Molina,* termed Quil A, and fractions thereof, described in US 5,057,540 and "Saponins as vaccine adjuvants", Kensil, C. R., Crit Rev Ther Drug Carrier Syst, 1996, 12 (1-2):1-55; and EP 0 362 279 B1. Particularly preferred fractions of Quil A are QS21, QS7, and QS17.

β-Escin is another preferred haemolytic saponins for use in the adjuvant compositions of the present invention. Escin is described in the Merck index (12^{th} ed: entry 3737) as a mixture of saponins occurring in the seed of the horse chestnut tree, Lat: *Aesculus hippocastanum.* Its isolation is described by chromatography and purification (Fiedler, Arzneimittel-Forsch. 4, 213 (1953)), and by ion-exchange resins (Erbring et al., US 3,238,190). Fractions of escin, α and β, have been purified and shown to be biologically active (Yoshikawa M, et al. (Chem Pharm Bull (Tokyo) 1996 Aug;44(8):1454-1464)). β-escin is also known as aescin.

Another preferred haemolytic saponin for use in the present invention is Digitonin. Digitonin is described in the Merck index (12^{th} Edition, entry 3204) as a saponin, being derived from the seeds of *Digitalis purpurea* and purified according to the procedure described Gisvold et al., J.Am.Pharm.Assoc., 1934, 23, 664; and Ruhenstroth-Bauer, Physiol. Chem., 1955, 301, 621. Its use is described as being a clinical reagent for cholesterol determination.

The adjuvant combinations of the present invention may further comprise a carrier, such that the saponin or CpG, or both, may be associated with a particulate carrier entity to enhance the adjuvanticity of the combination. Particularly preferred systemic vaccines, for example, comprise a carrier molecule.

The CpG used in the adjuvant combinations of the present invention may be in free solution or may be complexed to particulate carriers such as mineral salts (for example, but not restricted to, aluminium or calcium salts), liposomes, ISCOMs, emulsions (oil in water, water in oil, water in oil in water), polymers (such as, but not restricted to polylactic, polyglycolic, polyphosphazine, polyaminoacid, alginate, chitosan) or microparticles. Preferably said carriers are cationic. The vaccines of the present invention further comprise an antigen which may be associated with the CpG-carrier complex, or may not be associated with the CpG-carrier complex. In this case, the antigen may be free suspension or associated with a separate carrier.

The saponins forming part of the present invention may be separate in the form of micelles, or may be in the form of large ordered structures such as ISCOMs (EP 0 109 942 B1) or liposomes (WO 96/33739) when formulated with cholesterol and lipid, or in the form of an oil in water emulsion (WO 95/17210). The saponins may preferably be associated with a metallic salt, such as aluminium hydroxide or aluminium phosphate (WO 98/15287). Alternatively the saponin may be associated with a particulate carrier such as chitosan. The saponin may also be in a dry state such as a powder. The final formulations in the form as they are administered to the mucosal surface of the vaccinee are preferably haemolytic in nature. The saponin may or may not be associated physically with the antigen either through direct linkage or by co-interaction with the same particulate carrier molecule (GB9822712.7; WO 98/16247). The CpG and saponin in the adjuvants or vaccines of the present invention may themselves be separate or associated. For example, the CpG and saponin may be in free suspension or may be associated via a carrier, more preferably a particulate carrier such as aluminium hydroxide or by a cationic liposome or ISCOM.

A preferred adjuvant combination according to the present invention is composed of one or more CpG oligonucleotides containing at least 3, preferably at least 6 nucleotides between two adjacent CG motifs, together with QS21 and a particulate carrier selected from the group comprising an oil-in-water emulsion or DQ. Most preferably, the adjuvant combination comprises CpG 2006 (SEQ ID NO: 4), or CpG 1758 (SEQ ID NO: 2) or CpG 1826 (SEQ ID NO: 1) mixed with QS21, and a particulate carrier selected from the group comprising an oil-in-water emulsion or DQ. Accordingly, particularly preferred vaccines, for example, comprise such adjuvant combinations and an antigen. The preferred vaccine of the present invention is used to generate systemic immune responses after administration to an individual through the systemic route.

The adjuvant combinations of the present invention may be used as both systemic or mucosal adjuvant. In a particular form of the invention there is provided a systemic vaccine to be administered through the systemic or parenteral route such as intramuscular, intradermal, transdermal, subcutaneous, intraperitoneal or intravenous administration. A preferred route of administration is via the transdermal route, for example by skin patches.

The systemic vaccine preparations of the present invention may be used to protect or treat a mammal susceptible to, or suffering from disease, by means of administering said vaccine by intramuscular, intraperitoneal, intradermal, transdermal, intravenous, or subcutaneous administration. Methods of systemic administration of the vaccine preparations may include conventional syringes and needles, or devices designed for ballistic delivery of solid vaccines (WO 99/27961), or needleless pressure liquid jet device (US 4,596,556; US 5,993,412), or transdermal patches (WO 97/48440; WO 98/28037). The present invention may also be used to enhance the immunogenicity of antigens applied to the skin (transdermal or transcutaneous delivery WO 98/20734 ; WO 98/28037). The present invention therefore provides a delivery device for systemic administration, pre-filled with the vaccine or adjuvant compositions of the present invention. Accordingly there is provided a method for inducing an immune response in an individual, comprising the administration of a vaccine comprising an antigen and immunostimulatory oligonucleotide, a saponin, and a carrier, to the individual, wherein the vaccine is administered via the parenteral or systemic route. Preferred methods of inducing an immune response comprises the administration of a vaccine comprising an oligonucleotide of SEQ ID NO: 1, 2, 3, 4 or 5, with a saponin derived from Qui1A, such as QS21, and a carrier, such as an oil in water emulsion, a cholesterol containing liposome or alum.

Alternatively the vaccine preparations of the present invention may be used to protect or treat a mammal susceptible to, or suffering from disease, by means of administering said vaccine via a mucosal route, such as the oral/alimentary or nasal route. Alternative mucosal routes are intravaginal and intra-rectal. The preferred mucosal route of administration is via the nasal route, termed intranasal vaccination. Methods of intranasal vaccination are well known in the art, including the administration of a droplet, spray, or dry powdered form of the vaccine into the nasopharynx of the individual to be immunised. Nebulised or aerosolised vaccine formulations also form part of this invention. Enteric formulations such as gastro resistant capsules and granules for oral administration, suppositories for rectal or vaginal administration also form part of this invention.

The adjuvant combinations of the present invention, represent a class of mucosal adjuvants suitable for application in humans to replace systemic vaccination by mucosal vaccination. In a preferred form of the present invention pure saponins such as Quil A, or derivatives thereof, including QS21; Escin; Digitonin; or *Gypsophila* or *Chenopodium quinoa* saponins in combination with immunostimulatory oligonucleotides may be used as adjuvants for the mucosal administration of antigens to achieve a systemic immune response.

The adjuvant combinations of the present invention are used in the formulation of vaccines, which vaccines may be administered via the systemic or mucosal route. Preferably, when the vaccines are used for mucosal administration the adjuvant combination comprises a haemolytic saponin.

For mucosal administration preferably the composition of the invention comprise a haemolytic saponin. Haemolytic saponin, or saponin preparation, within the meaning of this invention is to be determined with reference to the following assay.
1. Fresh blood from guinea pigs is washed with phosphate buffered saline (PBS) 3 times in a desk-top centrifuge. After resuspension to the original volume the blood is further diluted 10 fold in PBS.
2. 50 µl of this blood suspension is added to 800 µl of PBS containing two-fold dilutions of surfactant or saponin.
3. After 8 hours the haemolysis is assessed visually or by measuring the optical density of the supernatant. The presence of a red supernatant, which absorbs light at 570 nm indicates the presence of haemolysis.
4. The results are expressed as the concentration of the first saponin dilution at which hemolysis no longer occurs.

For the purposes of this invention the saponin adjuvant preparation is haemolytic if it lyses the erythrocytes at a concentration of less than 0.1%. As means of reference, substantially pure samples of QuilA, QS21, QS7, Digitonin, and β-escin are all haemolytic saponins as defined in this assay.Within the inherent experimental variability of such a biological assay, the saponins of the present invention preferably have a haemolytic activity, of approximately between 0.5-0.00001%, more preferably between 0.05-0.00001%, even more preferably between 0.005-0.00001%, and most preferably between 0.001-0.0004%. Ideally, said saponins should have a haemolytic activity similar (*i.e*. within a ten-fold difference) to that of QS21.

The vaccines of the present invention may also be administered via the oral route. In such cases the pharmaceutically acceptable excipient may also include alkaline buffers, or enteric capsules or microgranules. The vaccines of the present invention may also be administered by the vaginal route. In such cases, the pharmaceutically acceptable excipients may also include emulsifiers, polymers such as CARBOPOL®, and other known stabilisers of vaginal creams and suppositories. The vaccines of the present invention may also be administered by the rectal route. In such cases the excipients may also include waxes and polymers known in the art for forming rectal suppositories.

Preparations of more than one saponin in the adjuvant combinations of the present invention are also form part of the present invention. For example combinations of at least two of the following group comprising QS21, QS7, Quil A, β-escin, or digitonin. Additionally, the compositions of the present invention may comprise combinations of more than one immunostimulatory oligonucleotide.

In a similar embodiment of the present invention the CpG/saponin combinations for both systemic and mucosal administration may be further combined with other adjuvants including. Monophosphoryl Lipid A and its non-toxic derivative 3-de-O-acylated monophosphoryl lipid A. Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

3 De-O-acylated monophosphoryl lipid A is a well known adjuvant manufactured by Ribi Immunochem, Montana. It can be prepared by the methods taught in GB 2122204B. A preferred form of 3 De-O-acylated monophosphoryl lipid A is in the form of an emulsion having a small particle size less than 0.2µm in diameter (EP 0 689 454 B1). Particularly preferred adjuvants are combinations of 3D-MPL and QS21 (EP 0 671 948 B1), oil in water emulsions comprising 3D-MPL and QS21 (WO 95/17210, WO 98/56414), or 3D-MPL formulated with other carriers (EP 0 689 454 B1).

Preferably the vaccine formulations of the present invention contain an antigen or antigenic composition capable of eliciting an immune response against a human pathogen, which antigen or antigenic composition is derived from HIV-1, (such as tat, nef, gp120 or gp160), human herpes viruses, such as gD or derivatives thereof or Immediate Early protein such as ICP27 from HSV 1 or HSV2, cytomegalovirus ((esp Human)(such as gB or derivatives thereof), Rotavirus (including live-attenuated viruses), Epstein Barr virus (such as gp350 or derivatives thereof), Varicella Zoster Virus (such as gpI, II and IE63), or from a hepatitis virus such as hepatitis B virus (for example Hepatitis B Surface antigen or a derivative thereof), hepatitis A virus, hepatitis C virus and hepatitis E virus, or from other viral pathogens, such as paramyxoviruses: Respiratory Syncytial virus (such as F and G proteins or derivatives thereof), parainfluenza virus, measles virus, mumps virus, human papilloma viruses (for example HPV6, 11, 16, 18, ..), flaviviruses (e.g. Yellow Fever Virus, Dengue Virus, Tick-borne encephalitis virus, Japanese Encephalitis Virus) or Influenza virus (whole live or inactivated virus, split influenza virus, grown in eggs or MDCK cells, or whole flu virosomes (as described by R. Gluck, Vaccine, 1992, 10, 915-920) or purified or recombinant proteins thereof, such as HA, NP, NA, or M proteins, or combinations thereof), or derived from bacterial pathogens such as *Neisseria spp,* including *N. gonorrhea* and *N. meningitidis* (for example capsular polysaccharides and conjugates thereof, transferrin-binding proteins, lactoferrin binding proteins, PilC, adhesins); *S. pyogenes* (for example M proteins or fragments thereof, C5A protease, lipoteichoic acids), *S. agalactiae, S. mutans; H. ducreyi; Moraxella spp, including M catarrhalis, also known as Branhamella* catarrhalis (for example high and low molecular weight adhesins and invasins); *Bordetella spp, including B. pertussis* (for example pertactin, pertussis toxin or derivatives thereof, filamenteous hemagglutinin, adenylate cyclase, fimbriae), *B. parapertussis and B. bronchiseptica; Mycobacterium spp., including M. tuberculosis* (for example ESAT6, Antigen 85A, -B or -C), *M. bovis, M. leprae, M. avium, M paratuberculosis, M. smegmatis; Legionella spp,* including *L. pneumophila; Escherichia spp,* including *enterotoxic E. coli* (for example colonization factors, heat-labile toxin or derivatives thereof, heat-stable toxin or derivatives thereof), enterohemorragic *E. coli,* enteropathogenic *E. coli* (for example shiga toxin-like toxin or derivatives thereof); *Vibrio spp,* including *V. cholera* (for example cholera toxin or derivatives thereof); *Shigella spp,* including *S*. *sonnei, S. dysenteriae, S. flexnerii; Yersinia spp,* including *Y*. enterocolitica (for example a Yop protein), *Y. pestis, Y. pseudotuberculosis; Campylobacter spp,* including *C. jejuni* (for example toxins, adhesins and invasins) and *C*. *coli; Salmonella spp,* including *S*. *typhi, S. paratyphi, S. choleraesuis, S. enteritidis; Listeria spp.,* including *L*. *monocytogenes; Helicobacter spp,* including *H*. pylori (for example urease, catalase, vacuolating toxin); *Pseudomonas spp,* including *P. aeruginosa; Staphylococcus spp.,* including *S*. *aureus, S. epidermidis; Enterococcus spp.,* including *E. faecalis, E. faecium; Clostridium spp.,* including *C*. tetani (for example tetanus toxin and derivative thereof), *C*. *botulinum* (for example botulinum toxin and derivative thereof), *C*. *difficile* (for example clostridium toxins A or B and derivatives thereof); *Bacillus spp.,* including *B. anthracis* (for example botulinum toxin and derivatives thereof); *Corynebacterium spp.,* including *C*. *diphtheriae* (for example diphtheria toxin and derivatives thereof); *Borrelia spp.,* including *B. burgdorferi* (for example OspA, OspC, DbpA, DbpB), *B. garinii* (for example OspA, OspC, DbpA, DbpB), *B. afzelii* (for example OspA, OspC, DbpA, DbpB), *B. andersonii* (for example OspA, OspC, DbpA, DbpB), *B. hermsii; Ehrlichia spp.,* including *E. equi* and the agent of the Human Granulocytic Ehrlichiosis; *Rickettsia spp,* including *R. rickettsii; Chlamydia spp., including C, trachomatis* (for example MOMP, heparin-binding proteins), *C. pneumoniae* (for example MOMP, heparin-binding proteins), *C. psittaci; Leptospira spp.,* including *L. interrogans; Treponema spp.,* including *T. pallidum* (for example the rare outer membrane proteins), *T. denticola, T. hyodysenteriae;* or derived from parasites such as *Plasmodium spp.,* including *P. falciparum; Toxoplasma spp.,* including *T. gondii (for example SAG2, SAG3, Tg34); Entamoeba spp.,* including *E. histolytica; Babesia spp.,* including *B. microti; Trypanosoma spp.,* including *T. cruzi; Giardia spp.,* including *G*. *lamblia; Leshmania spp.,* including *L. major; Pneumocystis spp.,* including *P. carinii; Trichomonas spp.,* including *T. vaginalis; Schisostoma spp.,* including *S*. *mansoni,* or derived from yeast such as *Candida spp.,* including *C*. *albicans; Cryptococcus spp.,* including *C*. *neoformans.*

Other preferred specific antigens for *M. tuberculosis* are for example Tb Ra12, Tb H9, Tb Ra35, Tb38-1, Erd 14, DPV, MTI, MSL, mTTC2 and hTCC1 (WO 99/51748). Proteins for *M. tuberculosis* also include fusion proteins and variants thereof where at least two, preferably three polypeptides of *M. tuberculosis* are fused into a larger protein. Preferred fusions include Ra12-TbH9-Ra35, Erd14-DPV-MTI, DPV-MTI-MSL, Erd14-DPV-MTI-MSL-mTCC2, Erd14-DPV-MTI-MSL, DPV-MTI-MSL-mTCC2, TbH9-DPV-MTI (WO 99/51748).

Most preferred antigens for Chlamydia include for example the High Molecular Weight Protein (HWMP) (WO 99/17741), ORF3 (EP 366 412), and putative membrane proteins (Pmps). Other Chlamydia antigens of the vaccine formulation can be selected from the group described in WO 99/28475.

Preferred bacterial vaccines comprise antigens derived from *Streptococcus spp,* including *S. pneumoniae* (for example capsular polysaccharides and conjugates thereof, PsaA, PspA, streptolysin, choline-binding proteins) and the protein antigen Pneumolysin (Biochem Biophys Acta, 1989, 67, 1007; Rubins et al., Microbial Pathogenesis, 25, 337-342), and mutant detoxified derivatives thereof (WO 90/06951; WO 99/03884). Other preferred bacterial vaccines comprise antigens derived from *Haemophilus spp., including H. influenzae type B* (for example PRP and conjugates thereof), *non typeable H. influenzae,* for example OMP26, high molecular weight adhesins, P5, P6, protein D and lipoprotein D, and fimbrin and fimbrin derived peptides (US 5,843,464) or multiple copy varients or fusion proteins thereof.

Derivatives of Hepatitis B Surface antigen are well known in the art and include, inter alia, those PreS1, PreS2 S antigens set forth described in European Patent applications EP-A-414 374; EP-A-0304 578, and EP 198-474. In one preferred aspect the vaccine formulation of the invention comprises the HIV-1 antigen, gp120, especially when expressed in CHO cells. In a further embodiment, the vaccine formulation of the invention comprises gD2t as hereinabove defined.

In a preferred embodiment of the present invention vaccines containing the claimed adjuvant comprise antigen derived from the Human Papilloma Virus (HPV) considered to be responsible for genital warts (HPV 6 or HPV 11 and others), and the HPV viruses responsible for cervical cancer (HPV16, HPV18 and others).

Particularly preferred forms of genital wart prophylactic, or therapeutic, vaccine comprise L1 particles or capsomers, and fusion proteins comprising one or more antigens selected from the HPV 6 and HPV 11 proteins E6, E7, L1, and L2.

The most preferred forms of fusion protein are: L2E7 as disclosed in WO 96/26277, and proteinD(1/3)-E7 disclosed in GB 9717953.5 (PCT/EP98/05285).

A preferred HPV cervical infection or cancer, prophylaxis or therapeutic vaccine, composition may comprise HPV 16 or 18 antigens. For example, L1 or L2 antigen monomers, or L1 or L2 antigens presented together as a virus like particle (VLP) or the L1 alone protein presented alone in a VLP or caposmer structure. Such antigens, virus like particles and capsomer are per se known. See for example WO94/00152, WO94/20137, WO94/05792, and WO93/02184.

Additional early proteins may be included alone or as fusion proteins such as E7, E2 or preferably E5 for example; particularly preferred embodiments of this includes a VLP comprising L1E7 fusion proteins (WO 96/11272).

Particularly preferred HPV 16 antigens comprise the early proteins E6 or E7 in fusion with a protein D carrier to form Protein D - E6 or E7 fusions from HPV 16, or combinations thereof; or combinations of E6 or E7 with L2 (WO 96/26277).

Alternatively the HPV 16 or 18 early proteins E6 and E7, may be presented in a single molecule, preferably a Protein D- E6/E7 fusion. Such vaccine may optionally contain either or both E6 and E7 proteins from HPV 18, preferably in the form of a Protein D - E6 or Protein D - E7 fusion protein or Protein D E6/E7 fusion protein.

The vaccine of the present invention may additionally comprise antigens from other HPV strains, preferably from strains HPV 31 or 33.

Vaccines of the present invention further comprise antigens derived from parasites that cause Malaria. For example, preferred antigens from *Plasmodia falciparum* include RTS,S and TRAP. RTS is a hybrid protein comprising substantially all the C-terminal portion of the circumsporozoite (CS) protein of *P.falciparum* linked via four amino acids of the preS2 portion of Hepatitis B surface antigen to the surface (S) antigen of hepatitis B virus. It's full structure is disclosed in the International Patent Application No. PCT/EP92/02591, published under Number WO 93/10152 claiming priority from UK patent application No.9124390.7. When expressed in yeast RTS is produced as a lipoprotein particle, and when it is co-expressed with the S antigen from HBV it produces a mixed particle known as RTS,S. TRAP antigens are described in the International Patent Application No. PCT/GB89/00895, published under WO 90/01496. A preferred embodiment of the present invention is a Malaria vaccine wherein the antigenic preparation comprises a combination of the RTS,S and TRAP antigens. Other plasmodia antigens that are likely candidates to be components of a multistage Malaria vaccine are *P. faciparum* MSP1, AMA1, MSP3, EBA, GLURP, RAP1, RAP2, Sequestrin, PfEMP1, Pf332, LSA1, LSA3, STARP, SALSA, PfEXP1, Pfs25, Pfs28, PFS27/25, Pfs16, Pfs48/45, Pfs230 and their analogues in Plasmodium spp.

The formulations may also contain an anti-tumour antigen and be useful for the immunotherapeutic treatment of cancers. For example, the adjuvant formulation finds utility with tumour rejection antigens such as those for prostrate, breast, colorectal, lung, pancreatic, renal or melanoma cancers. Exemplary antigens include MAGE 1 and MAGE 3 or other MAGE antigens (for the treatment of melanoma), PRAME, BAGE, or GAGE (Robbins and Kawakami, 1996, Current Opinions in Immunology 8, pps 628-636; Van den Eynde et al., International Journal of Clinical & Laboratory Research (submitted 1997); Correale et al. (1997), Journal of the National Cancer Institute 89, p293. Indeed these antigens are expressed in a wide range of tumour types such as melanoma, lung carcinoma, sarcoma and bladder carcinoma. Other tumour-specific antigens are suitable for use with the adjuvants of the present invention and include, but are not restricted to tumour-specific gangliosides, Prostate specific antigen (PSA) or Her-2/neu, KSA (GA733), PAP, mammaglobin, MUC-1, carcinoembryonic antigen (CEA). Accordingly in one aspect of the present invention there is provided a vaccine comprising an adjuvant composition according to the invention and a tumour rejection antigen.

It is a particularly preferred aspect of the present invention that the vaccines comprise a tumour antigen; such vaccines are surprisingly potent in the therapy of cancer such as prostrate, breast, colorectal, lung, pancreatic, renal, ovarian or melanoma cancers. Accordingly, the formulations may contain tumour-associated antigen, as well as antigens associated with tumour-support mechanisms (e.g. angiogenesis, tumour invasion). Additionally, antigens particularly relevant for vaccines in the therapy of cancer also comprise Prostate-specific membrane antigen (PSMA), Prostate Stem Cell Antigen (PSCA), tyrosinase, survivin, NY-ESO1, prostase, PS108 (WO 98/50567), RAGE, LAGE, HAGE. Additionally said antigen may be a self peptide hormone such as whole length Gonadotrophin hormone releasing hormone (GnRH, WO 95/20600), a short 10 amino acid long peptide, useful in the treatment of many cancers, or in immunocastration.

It is foreseen that compositions of the present invention will be used to formulate vaccines containing antigens derived from *Borrelia sp..* For example, antigens may include nucleic acid, pathogen derived antigen or antigenic preparations, recombinantly produced protein or peptides, and chimeric fusion proteins. In particular the antigen is OspA. The OspA may be a full mature protein in a lipidated form virtue of the host cell (E.Coli) termed (Lipo-OspA) or a non-lipidated derivative. Such non-lipidated derivatives include the non-lipidated NS1-OspA fusion protein which has the first 81 N-terminal amino acids of the non-structural protein (NS1) of the influenza virus, and the complete OspA protein, and another, MDP-OspA is a non-lipidated form of OspA carrying 3 additional N-terminal amino acids.

Vaccines of the present invention may be used for the prophylaxis or therapy of allergy. Such vaccines would comprise allergen specific (for example Der p1) and allergen non-specific antigens (for example peptides derived from human IgE, including but not restricted to the stanworth decapeptide (EP 0 477 231 B1)).

Vaccines of the present invention may also be used for the prophylaxis or therapy of chronic disorders others than allergy, cancer or infectious diseases. Such chronic disorders are diseases such as atherosclerosis, and Alzheimer.

Antigens relevant for the prophylaxis and the therapy of patients susceptible to or suffering from Alzheimer neurodegenerative disease are, in particular, the N terminal 39 -43 amino acid fragment (Aβ) of the amyloid precursor protein and smaller fragments. This antigen is disclosed in the International Patent Application No. WO 99/27944 - (Athena Neurosciences).

The amount of protein in each vaccine dose is selected as an amount which induces an immunoprotective response without significant, adverse side effects in typical vaccinees. Such amount will vary depending upon which specific immunogen is employed and how it is presented. Generally, it is expected that each dose will comprise 1-1000 µg of protein, preferably 1-500 µg, preferably 1-100µg, most preferably 1 to 50µg. An optimal amount for a particular vaccine can be ascertained by standard studies involving observation of appropriate immune responses in vaccinated subjects. Following an initial vaccination, subjects may receive one or several booster immunisation adequately spaced. Such a vaccine formulation may be applied to a mucosal surface of a mammal in either a priming or boosting vaccination regime; or alternatively be administered systemically, for example *via* the transdermal, subcutaneous or intramuscular routes.

The amount of CpG or immunostimulatory oligonucleotides in the adjuvants or vaccines of the present invention is generally small, but depending on the vaccine formulation may be in the region of 1-1000µg per dose, preferably 1-500µg per dose, and more preferably between 1 to 100µg per dose.

The amount of saponin for use in the adjuvants of the present invention may be in the region of 1-1000µg per dose, preferably 1-500µg per dose, more preferably 1-250µg per dose, and most preferably between 1 to 100µg per dose. The ratio of CpG:saponin (w/w) will, therefore, be in the range of 1:1000 to 1000:1, and will typically be in the range of 1:100 to 100:1, and preferably in the range of 1:10 to 1:1 or 1:1 to 10:1, and most preferably 1:1, 4:1 or 10:1.

The formulations of the present invention maybe used for both prophylactic and therapeutic purposes. Accordingly, there is provided the use of a combination of a saponin and a CpG molecule in the manufacture of a vaccine for the prophylaxis and the treatment of viral, bacterial, parasitic infections, allergy, cancer and other non-chronic disorders. Accordingly, the present invention provides for a method of treating a mammal susceptible to or suffering from an infectious disease or cancer, or allergy, or autoimmune disease. In a further aspect of the present invention there is provided a vaccine or adjuvant combination, comprising a saponin and CpG, as herein described for use as a medicament. Vaccine preparation is generally described in New Trends and Developments in Vaccines, edited by Voller et al., University Park Press, Baltimore, Maryland, U.S.A. 1978.

It is foreseen that compositions of the present invention will be used to formulate vaccines containing antigens derived from a wide variety of sources. For example, antigens may include human, bacterial, or viral nucleic acid, pathogen derived antigen or antigenic preparations, tumour derived antigen or antigenic preparations, host-derived antigens, including peptides derived from IgE, such as the histamine releasing decapeptide of IgE (known as the Stanworth decapeptide), recombinantly produced protein or peptides, and chimeric fusion proteins.

There is provided by the present invention a systemic vaccine composition comprising an antigen, a saponin and an immunostimulatory oligonucleotide. Accordingly, there is provided a method of treatment of an individual susceptible to or suffering from a disease by the administration of a composition as substantially described herein through the systemic route of said individual. Also provided is a method to prevent an individual from contracting a disease selected from the group comprising infectious bacterial and viral diseases, parasitic diseases, prostate, breast, colorectal, lung, pancreatic, renal, ovarian or melanoma cancers; non-cancer chronic disorders, allergy, Alzheimer, atherosclerosis, comprising the administration of a composition as substantially described herein through the systemic route of said individual.

Alternatively, there is provided by the present invention a mucosal vaccine composition comprising an antigen, and a haemolytic saponin. Accordingly, there is provided a method of treatment of an individual susceptible to or suffering from a disease by the administration of a composition as substantially herein described to a mucosal surface of said individual.

Furthermore, there is described a method of inducing a systemic antigen specific immune response in a mammal, comprising administering to a mucosal surface of said mammal a composition comprising an antigen and a haemolytic saponin. Further there is provided a method of manufacture of a vaccine or adjuvant are also provided, comprising taking a saponin and taking a CpG molecule and admixing them with an antigen.

Examples of suitable pharmaceutically acceptable excipients for use in the combinations of the present invention include water, phosphate buffered saline, isotonic buffer solutions.

### FIGURE LEGENDS

Figure 1: OspA specific IgG titres 14 days after the nasal boosting.
Figure 2: OspA specific LA2 titres 14 days after the nasal boosting.
Figure 3: serum Flu strain specific IgG titres 14 days after the nasal boosting.
Figure 4: serum Flu strain specific serum HemAgglutination Inhibition (HAI) titres 14 days after the nasal boosting.
Figure 5: OspA specific LA2 titres in mice
Figure 6: gp120-specific lymphoproliferation activity of spleen cells from immunized mice. The antigen-specific activity is expressed as SI for different antigen concentrations for all 4 experimental groups.
Figure 7: HBsAg-specific CTL activity of spleen cells from immunized mice. Effector cell activity was assessed by examining ⁵¹Cr release of P815 cells (open circles) or *s-*transfected P815 cells (closed circles).
Figure 8: HBsAg-specific antibody responses in immunized mice. Specific antibody titers (expressed as EU/ml) and isotype profiles were evaluated using ELISA tests. Values from pooled sera are shown in the table, and isotype distributions are also depicted in a graphic.
Figure 9: HBSAg- and gp120-specific lymphoproliferation activity of spleen cells from immunized mice. The antigen-specific activity is expressed as SI for different antigen concentrations for all 4 experimental groups.
Figure 10: HBsAg- and gp 120-specific CTL activity of spleen cells from immunized mice. Effector cell activity was assessed by examining ⁵¹Cr release of control P815 cells (open symbols) or P815 cells displaying an HBsAg or gp120 CTL epitope (closed symbols).
Figure 11: Gp 120-specific and HbsAg-specific antibody responses in immunized mice. Specific antibody titers (expressed in µg/ml) (Figure 11A) and isotype profiles were evaluated using ELISA tests. Values from pooled sera are shown in the table, and isotype distributions are also depicted in a graphic. Figure 11B shows the isotype pattern of gp120-specific antibodies.
Figure 12: Evolution of the mean tumour growth per groups of 10 animals over time.

The present invention is illustrated by, but not restricted to, the following examples.

### EXAMPLE 1 The use of QS21 and CpG for the intranasal boosting of systemic antibodies to Lipo-OspA

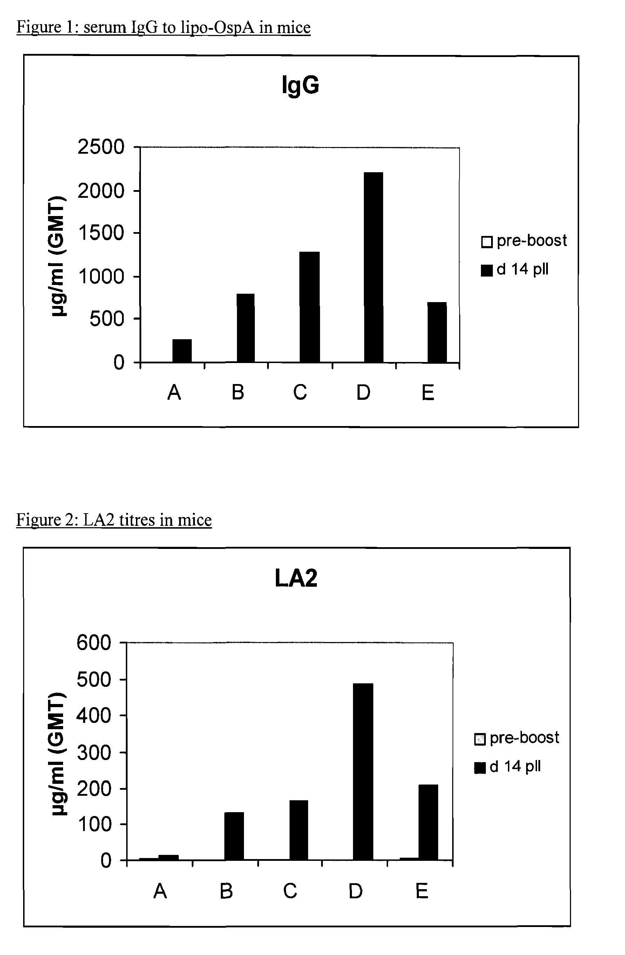

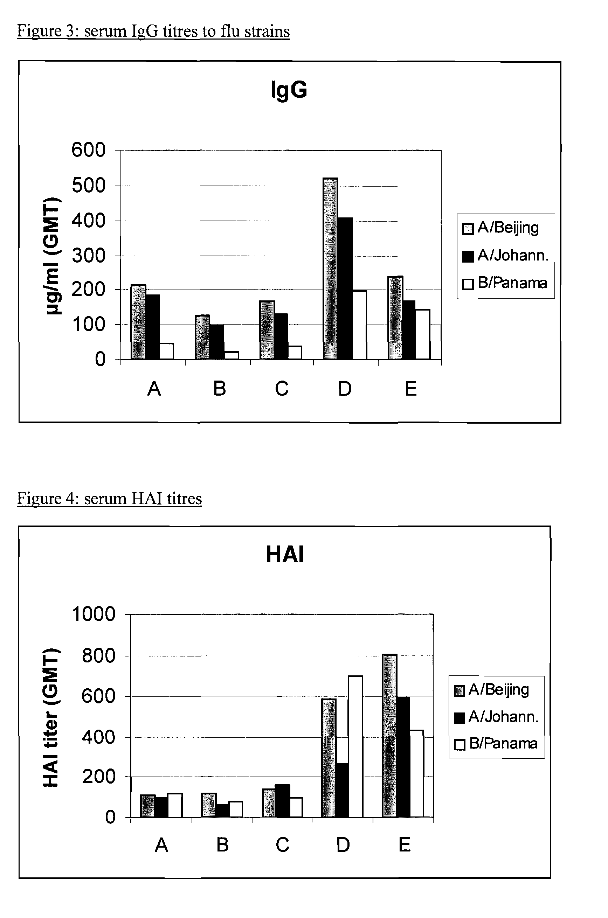

In this example we investigated whether lytic saponins such as QS21 and immunostimulants such as CpG were able to enhance in a synergistic fashion systemic immunological responses to an intranasal boosting vaccination of mice. Female Balb/c mice (5 animals per group), aged 8 weeks, were immunized intramuscularly with lipo-OspA (1µg) formulated onto alum (50 µg). After 3 months, the mice were boosted intranasally (under anesthesia) with 10 µl of solution (5 µl per nostril, delivered as droplets by pipette) containing 5 µg lipo-OspA in either A: PBS; B: 20 µg CpG 1001 (TCC ATG AGC TTC CTG ACG TT, Krieg 1826); C: 5 µg QS21 (obtained from Cambridge Biotech, USA); D: 20 µg CpG 1001 + 5 µg QS21; or, E: by intra muscular injection of 1 µg lipo-OspA adsorbed onto alum (50 µg). Figures 1 and 2 show the OspA specific IgG titres and LA2 titres 14 days after the nasal boosting.

### Methods

### ELISA for the measurement of OspA-specific serum IgG in mice:

Maxisorp Nunc immunoplates are coated overnight at 4°C with 50 µl/well of 1 µg/ml OspA diluted in PBS (in rows B to H of plate), or with 50 µl of 5 µg/ml purified goat anti-mouse Ig (Boerhinger), in PBS (row A). Free sites on the plates are blocked (1 hour, 37°C) using saturation buffer : PBS containing 1%BSA, 0.1% polyoxyethylene sorbitan monolaurate (TWEEN 20), and 4% Normal Bovine Serum (NBS). Then, serial 2-fold dilutions of IgG isotype mixture, diluted in saturation buffer (50 µl per well) and added as a standard curve (mixture of mouse monoclonal antibodies IgG1, IgG2a and IgG2b from Sigma, starting at 200 ng/ml and put in row A), and serum samples (starting at a 1/100 dilution and put in rows B to H) are incubated for 1hr 30mins at 37°C. The plates are then washed (x3) with washing buffer (PBS, 0.1% polyoxyethylene sorbitan monolaurate (TWEEN 20)). Then, biotinylated goat anti-mouse IgG (Amersham) diluted 1/5000 in saturation buffer are incubated (50 µl/well) for 1hr 30mins, at 37°C. After 3 washings, and subsequent addition of streptavidin-horseradish peroxidase conjugate (Amersham), plates are washed 5 times and incubated for 20 min at room temperature with 50 µl/well of revelation buffer (OPDA 0.4 mg/ml (Sigma) and H₂O₂ 0.03% in 50mM pH 4.5 citrate buffer). Revelation is stopped by adding 50 µl/well H₂SO₄ 2N. Optical densities are read at 492 and 630 nm by using Biorad 3550 immunoreader. Antibody titers are calculated by the 4 parameter mathematical method using SoftMaxPro software.

### Inhibition assay for the measurement of serum LA2-like Antibody titres to lipo-OspA

Antibody titres in the vaccinees were studied with respect to their LA2-like specificity. LA2 is a murine monoclonal antibody which recognizes a conformational OspA epitope at the surface of the bacteria and has been shown to be able to kill B. burgdorferi in vitro, as well as to protect mice against a challenge with laboratory-grown spirochete (Schaible UE et al. 1990. Proc Natl Acad Sci USA 87:3768-3772). Moreover, LA-2 mab has been shown to correlate with bactericidal antibodies, and studies on human sera showed also a good correlation between the total anti-OspA IgG titers and the LA-2 titers (as measured by ELISA).
Maxisorp Nunc immunoplates are coated overnight at 4°C with 50 µl/well of 0.5µg/ml lipo OspA diluted in PBS. Free sites were blocked with saturation buffer for 1hr at 37°C with (100 µl/well of saturation buffer: PBS/ BSA 1%/ Tween 20 0.1%/ NBS 4%). Serial 2-fold dilutions of LA2 monoclonal Ab (mAb) starting at 4 µg/ml were diluted in saturation buffer (50 µl per well) to form a standard curve. Dilutions of serum samples from the vaccinees (starting at a 1/10 dilution) were also added and the plates incubated for 2hrs at 37°C. The plates were washed after incubation 3 times with PBS/ TWEEN 20 (0.1%). LA2 mAb-peroxidase conjugate (1/10,000) diluted in saturation buffer was added to each well (50 µl/well) and incubated for 1hr at 37°C. After 5 washings, plates are incubated for 20 min at room temperature (in darkness) with 50 µl/well of revelation buffer (OPDA 0.4 mg/ml and H₂O₂ 0.03% in 50mM pH 4.5 citrate buffer). The reaction and colour formation was stopped with H₂SO₄2N. Optical densities are read at 492 and 630 nm by using Biorad 3550 immunoreader. LA2-like Ab titers are calculated by the 4 parameter mathematical method using SoftMaxPro software. LA2-like antibody titres were determined by comparison with the standard curve.

### Results

CpG as well as QS21 improve significantly the intranasal boosting of systemic antibodies to Lipo-OspA. Moreover, when both adjuvants are combined, a synergistic effect on those responses is clearly demonstrated, especially in term of LA2 antibodies. Humoral responses elicited in the presence of QS21 and CpG are significantly higher than those induced by the parenteral booster. Taken together, these results show clearly the potential of intranasal formulations combining a lytic saponin and an immunostimulant.

### EXAMPLE 2. Synergistic combination of QS21 and CpG for enhancing the intranasal boosting of systemic antibodies to influenza virus

In this example we investigated whether haemolytic saponins such as QS21 (see example ) and immunostimulants such as CpG were able to enhance in a synergistic fashion the intranasal boost of systemic antibodies in mice primed intranasally with inactivated whole influenza virus.
Female Balb/c mice (10 animals per group), aged 8 weeks, were primed intranasally with β-propiolactone inactivated trivalent whole influenza virus (A/Beijing/262/95; A/Johannesburg/33/94; B/Panama/45/90; 5 µg HA / strain) for mimicking the natural priming occurring in humans. After 28 days, the mice were boosted intranasally (under anesthesia) with 20 µl of solution (10 µl per nostril, delivered as droplets by pipette) containing 1.5 µg HA / strain of β-propiolactone inactivated trivalent whole influenza virus (same strains as in the priming immunization) in either A: PBS; B: 50 µg CpG (TCG TCG TTT TGT CGT TTT GTC GTT, Krieg 2006); C: 4.5 µg QS21 (obtained from Cambridge Biotech, USA); D: 50 µg CpG + 4.5 µg QS21; or, E: by intra muscular injection of 1.5 µg HA / strain of trivalent split influenza virus (same strains as in the priming immunization). Flu antigens were supplied by SSD GmBH manufacturer (Dresden, Germany).
Figures 3 and 4 show the serum Flu strain specific IgG titres and HemAgglutination Inhibition (HAI) titres 14 days after the nasal boosting.

### Methods

### ELISA for the measurement of Anti-influenza IgG titres in mice:

Maxisorp Nunc immunoplates are coated overnight at 4°C with 50 µl/well of 1 µg/ml whole influenza virus antigen diluted in PBS (in rows B to H of plate), or with 50 µl of 5 µg/ml purified goat anti-mouse Ig (Boerhinger), in PBS (row A). Free sites on the plates are blocked (1 hour, 37°C) using saturation buffer : PBS containing 1%BSA, 0.1% polyoxyethylene sorbitan monolaurate (TWEEN 20), and 4% Normal Bovine Serum (NBS). Then, serial 2-fold dilutions of IgG isotype mixture, diluted in saturation buffer (50 µl per well) and added as a standard curve (mixture of mouse monoclonal antibodies IgG1, IgG2a and IgG2b from Sigma, starting at 200 ng/ml and put in row A), and serum samples (starting at a 1/100 dilution and put in rows B to H) are incubated for 1hr 30mins at 37°C. The plates are then washed (x3) with washing buffer (PBS, 0.1% polyoxyethylene sorbitan monolaurate (TWEEN 20)). Then, biotinylated goat anti-mouse IgG (Amersham) diluted 1/5000 in saturation buffer are incubated (50 µl/well) for 1hr 30mins, at 37°C. After 3 washings, and subsequent addition of streptavidin-horseradish peroxidase conjugate (Amersham), plates are washed 5 times and incubated for 20 min at room temperature with 50 µl/well of revelation buffer (OPDA 0.4 mg/ml (Sigma) and H₂O₂ 0.03% in 50mM pH 4.5 citrate buffer). Revelation is stopped by adding 50 µl/well H₂SO₄2N. Optical densities are read at 492 and 630 nm by using Biorad 3550 immunoreader. Antibody titers are calculated by the 4 parameter mathematical method using SoftMaxPro software. The Whole influenza virus used for the coating (strain A/Beijing/262/95), inactivated with β-propiolactone (BPL), is supplied by SSD GmBH manufacturer (Dresden, Germany).

### HemAgglutination Inhibition (HAI) activity of Flu-specific serum Abs in mice

Sera (25 µl) are first treated for 20 minutes at room temperature (RT) with 100 µl borate 0.5M buffer (pH 9) and 125 µl Dade Behring-purchased kaolin. After centrifugation (30 minutes, 3000 RPM or 860 g), 100 µl supernatant (corresponding to a 1/10 dilution of the serum) are taken and incubated for 1 hour at 4°C with 0.5% chicken red blood cells. Supernatant is collected after centrifugation for 10 minutes at 3200 RPM (970 g). Both operations are done for eliminating the natural hemagglutinating factors contained in the sera. Then, 25 µl treated-sera are diluted in 25 µl PBS (serial 2-fold dilutions starting at 1/20) in 96 well Greiner plates. BPL inactivated whole virus is added (25 µl / well) at a concentration of 4 Hemagglutination Units (i.e. at a dilution which is 4-fold lower than the last one provoking an agglutination of red blood cells) for 30 minutes at RT under agitation. Chicken red blood cells are then added (25 µl / well) for 1 hour at RT. Plates are finally kept overnight at 4°C before to be read. The HAI titer corresponds to the last serum dilution inhibiting the virus-induced hemagglutination.

### Results

CpG as well as QS21 do not improve the intranasal boosting of IgG or HAI antibodies to Flu strains. However, when both adjuvants are combined, a synergistic effect on those responses is clearly demonstrated. The HAI responses elicited in the presence of QS21 and CpG are even similar than those induced by the parenteral booster. These results confirm the potential of intranasal formulations combining a haemolytic saponin and an immunostimulant. They also show that several CpG sequences can be efficient in this context (Krieg 2006 in the present example and Krieg 1826 in the examples 3 and 5).

### EXAMPLE 3. Synergistic combination of β-Escin and CpG for enhancing the intranasal boosting of systemic antibodies to Lipo-OspA

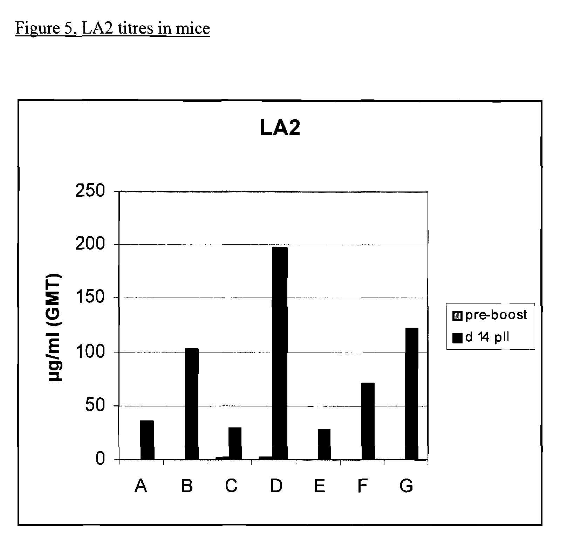

We assess in the present example the possibility that a synergy similar to that observed between QS21 and CpG could be obtained with other haemolytic saponins (see example) such as β-Escin. The non haemolytic saponin, glycyrrhizic acid, is also tested.
Female Balb/c mice (6 animals per group), aged 8 weeks, were primed intramuscularly with lipo-OspA (1µg) formulated onto alum (50 µg). After 3 months, the mice were boosted intranasally (under anesthesia) with 10 µl of solution (5 µl per nostril, delivered as droplets by pipette) containing 5 µg lipo-OspA in either A: PBS; B: 50 µg CpG 1001 (TCC ATG AGC TTC CTG ACG TT, Krieg 1826); C: 5 µg β-Escin (purchased from Sigma); D: 50 µg CpG 1001 + 5 µg β-Escin; E: 5 µg glycyrrhizic acid (purchased from Sigma); F: 50 µg CpG 1001 + 5 µg glycyrrhizic acid or, G: by intra muscular injection of 1 µg lipo-OspA adsorbed onto alum (50 µg). Figure 5 shows the OspA specific-LA2 titres 14 days after the nasal boosting.

### Methods

The methods are the same as those detailed in Example 1.

### Results

β-Escin and CpG act synergistically for enhancing the intranasal boosting of systemic LA2 Abs. This combination elicits more elevated Ab responses than the parenteral booster. On the other hand, such a synergy is not obtained by combining CpG with glycyrrhizic acid.

These results and the previous ones of this patent taken together show the ability of CpG and different haemolytic saponins to adjuvant immune responses in a synergistic fashion.

### EXAMPLE 4. Immunogenicity studies using P. falciparum RTS,S and HIV-1 gp120 formulated with CpG and/or DQS21

### 1. Experiment outline

Two mouse immunogenicity studies were conducted to evaluate potential additive or synergistic effects of CpG oligonucleotides (CpG) and QS21. Groups of mice were immunized with RTS,S and gp120 formulated with CpG and QS21 alone or in combination. These adjuvant combinations were also tested in the presence of the carrier Al(OH)₃ or an oil-in-water (o/w) emulsion.

The immunogenicity of the formulations was examined after two parenteral immunizations. Sera were analyzed for the presence of antigen-specific antibodies, and for the distribution of antibody isotypes. Spleen cells were used to evaluate cell-mediated immune responses. Those cells were tested for the presence of cytotoxic T lymphocytes (CTL) and lymphoproliferative (lymphoproliferation) cells.

**Table 1: Groups of mice in experiment 1**

| **Group** | **antigen** | **adjuvant** |
|---|---|---|
| 1 | RTS,S/gp120 | CpG/DQS21 |
| 2 | RTS,S/gp120 | DQS21 |
| 3 | RTS,S/gp120 | CpG/DQS21/Al(OH)₃ |
| 4 | RTS,S/gp120 | CpG/Al(OH)₃ |

**Table 2: Groups of mice in experiment 2**

| **Group** | **antigen** | **adjuvant** |
|---|---|---|
| 1 | RTS,S/gp120 | CpG |
| 2 | RTS,S/gp120 | CpG/DQS21 |
| 3 | RTS,S/gp120 | CpG/QS21/o/w emulsion |

### 2. Formulation

### 2.1. Experiment 1

### Formulation process:

Formulations were prepared three days before each injection. When needed, RTS,S (10 µg) and gp 120 (10µg) were adsorbed on 100 µg of AL(OH)₃. When needed, MPL (5 µg) was added and incubated 30 min before buffer addition as a mix of 10-fold concentrated PBS pH 7.4 and H₂O excepted for the group without DQ for which the buffer was PO₄, NaCl 10/150 pH 6.8. After 30 min, if needed, QS21 (5 µg) mixed with liposomes in a weight ratio QS21/cholesterol of 1/5 (referred to as DQ) was added to the formulation. Thirty minutes later, for the formulations with the oligo, 100 µg of CpG was added 30 min prior addition of 50 µg/ml of thiomersal as preservative.

| |
|---|
| Al(OH)₃ + RTSS + gp120 -1h- MPL -30min- premix -30min- DQ -30min- CpG -30min- Thio |

All incubations were carried out at room temperature with agitation.

### 2.2. Experiment 2

### Formulation process:

Formulations are performed simultaneously for both injections. The volume of injection for one mouse is 100 µl. Fifty µg/ml of thiomersal is added as preservative.
Group 1 : RTS,S (10 µg) and gp 120 (10 µg) are diluted with H₂O and PBS pH 6.8 for isotonicity. After 5 min., the formulation is adsorbed on CpG 1856 (100 µg).
Group 2 : RTS,S (10 µg) and gp120 (10 µg) are diluted with H₂O and PBS pH 7.4 for isotonicity. After 30 minutes RTS,S and gp120 are adsorbed on DQ (5 µg) . After 30 min. of adsorption, the formulation is adsorbed on CpG 1856 (100 µg).
Group 3 : RTS,S (10 µg) and gp120 (10 µg) are diluted with H₂O and PBS pH 6.8 for isotonicity. After 5 min., the formulation is adsorbed on an o/w emulsion. After 5 min. of adsorption, the formulation is adsorbed on QS21 (5 µg) prior the addition of CpG (100 µg).

### 3. Immunological methods

Nine (Balb/C x C57B1/6) F1 mice per group received into the hind footpads 2 x 50 µl vaccine twice at a two-week-interval. Two weeks later sera were obtained to assess antibody responses, and spleen cells were harvested to determine cell-mediated immune responses.

For lymphoproliferation analysis, cells were seeded in quadruplicates in 96-well round-bottomed microtiter plates at a concentration of 2x10⁶ per ml. Cells were cultured for 72 or 96 hrs in RPMI-1640 supplemented with antibiotics, glutamine and 1 % (v/v) normal mouse serum in the presence of different concentrations of RTS,S or gp120 antigen. Control cells were cultured without antigen. Then the cells were pulsed overnight with 1 µCi/well [³H]-thymidine, harvested and the incorporated radioactivity was determined in a beta-counter. Results are expressed as mean counts per minute (cpm).

For CTL analysis cells were cultured for 7 days in 6-well plates in the presence of 10 µg per ml of synthetic peptide pCMI003 (IPQSLDSWWTSL) corresponding to an HBsAg CTL epitope (Schirmbeck et al., 1995) or peptide pCMI007 (GIHIGPGRAFYAARK) representing an gp120 CTL epitope (Casement et al., 1995). At the end of the culture period effector cells were assessed in duplicate for HBsAg-specific cytolytic activity in standard [⁵¹Cr]-release assays using control and S-transfected P815 cells. Gp120-specific cytotoxicity was determined by using P815 target cells that were either left untreated or pulsed for 1 hr with peptide pCMI007. Minimum and maximum release were determined with target cells without effector cells and by the addition of 3 % (v/v) Triton X-100, respectively. Results are expressed as % [51Cr]-release (cpm of experimental culture - cpm of spontaneous release / cpm of maximum release - cpm of spontaneous release).

Titration and isotyping of pooled sera was performed in a standard enzyme-linked immunosorbent assay (ELISA) format using plates coated with HbsAg. Sera were diluted in PBS/BSA starting at 1:400. Biotinylated secondary antibodies specific for Ig or the isotypes IgG1, IgG2a and IgG2b followed by a horseradish peroxydase-streptavidin conjugate were used for detection of bound antibodies. ELISA titers were calculated from a reference by SoftmaxPro and expressed in ELISA units (EU/ml). Gp120-specific antibody titers were determined in a standard ELISA using plates coated with gp120 protein. Sera were diluted in PBS/Tween20BSA starting at 1:100. Biotinylated secondary antibodies specific for Ig or the isotypes IgG1, IgG2a and IgG2b followed by a horseradish peroxydase-streptavidin conjugate were used for detection of bound antibodies. Titers were calculated in relative to a standard mouse Ig and expressed as µg/ml.

### 4. Results

### Experiment 1

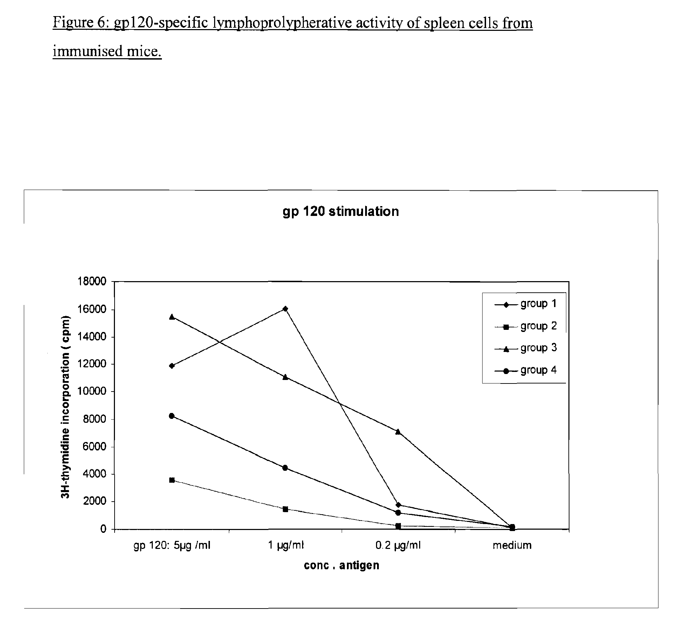

Analysis of lymphoproliferation responses did not show any significant differences in reactivity to RTS,S between the groups. In contrast, the groups 1 and 3 containing both, CpG and DQS21, showed better gp 120-specific Lymphoprolypheration responses than the groups containing CpG or DQS21 alone (Figure 6).

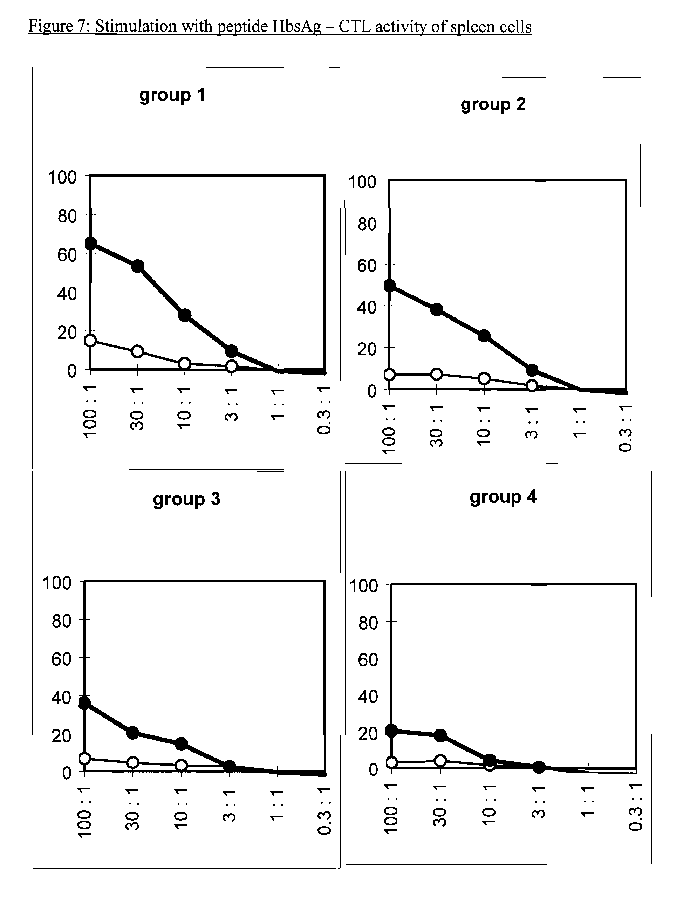

In this experiment only HBsAg-specific CTL were measured. There was no pronounced difference in CTL induction between the groups 1 and 3 having received CpG and DQS21 in combination and the groups 2 and 4 immunized with only one of the two adjuvant components, while the presence of Al(OH)₃ diminished the CTL activity observed for the combination of CpG and DQS21 in group 1 (Figure 7). However, a trend was present that CpG and DQS21 was better than DQS21 alone, and the combination induced more CTL in the presence of Al(OH)₃ than CpG alone (Figure 7).

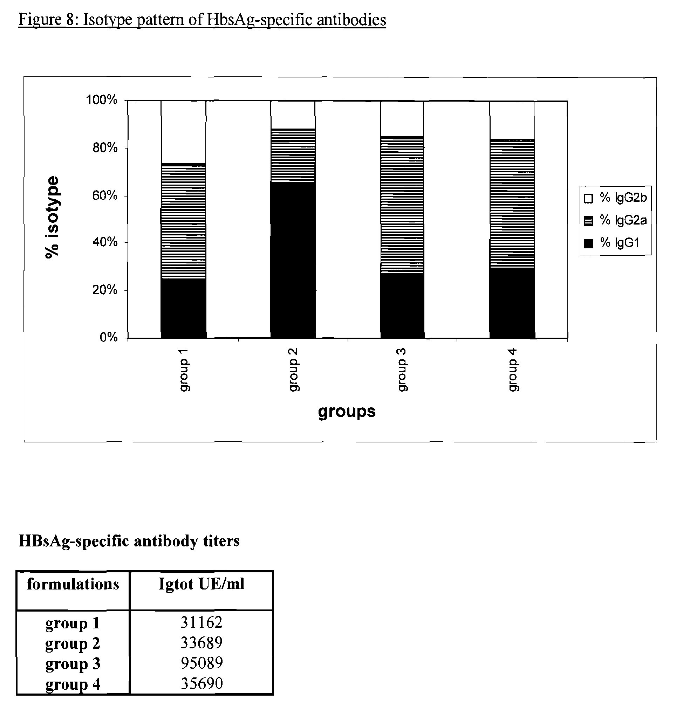

The humoral immune response of the mice was examined only for the presence of HBsAg-specific antibodies. Titers were similar in all groups expect for group 3, which showed an approximately three-fold increase, demonstrating that, in the presence of Al(OH)₃, the combination of DQS21 and CpG is more immunogenic than CpG alone (Figure 8). The isotype distribution was similar for the Al(OH)₃-containing groups 3 and 4, while in the absence of Al(OH)₃ the combination of CpG and DQS21 induced a stronger T_{H1}-like isotype pattern than DQS21 alone (Figure 8).

### Experiment 2

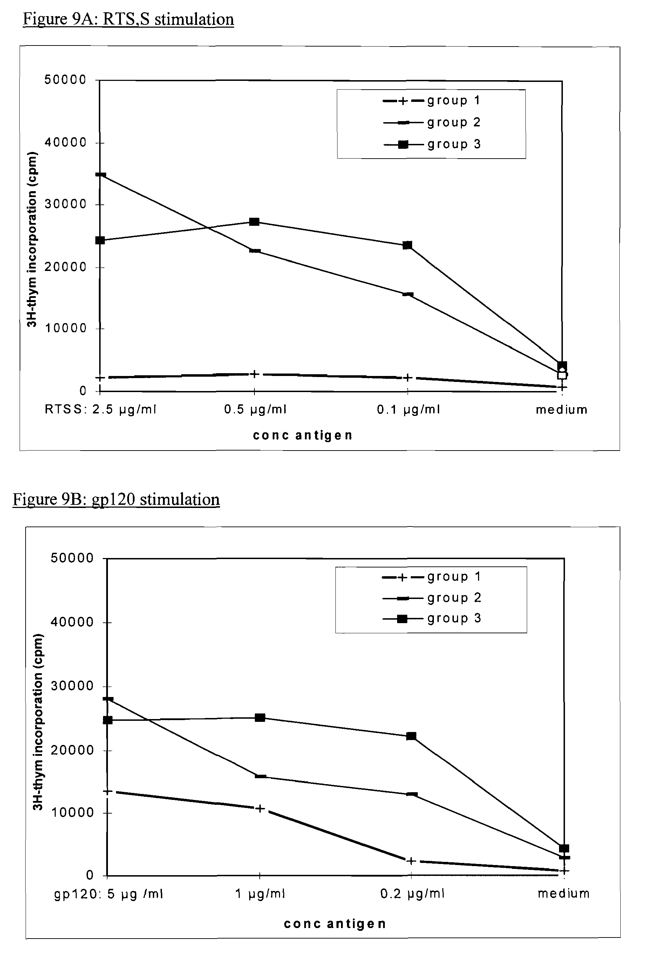

Lymphoproliferation responses specific for RTS,S and gp120 were very similar in this experiment. The data indicate that the addition of DQS21 (either alone or with an o/w emulsion) enhances lymphoproliferation responses to both antigens (Figure 9).

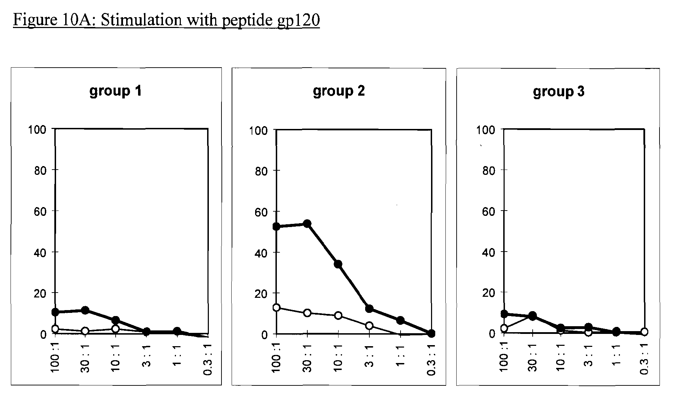

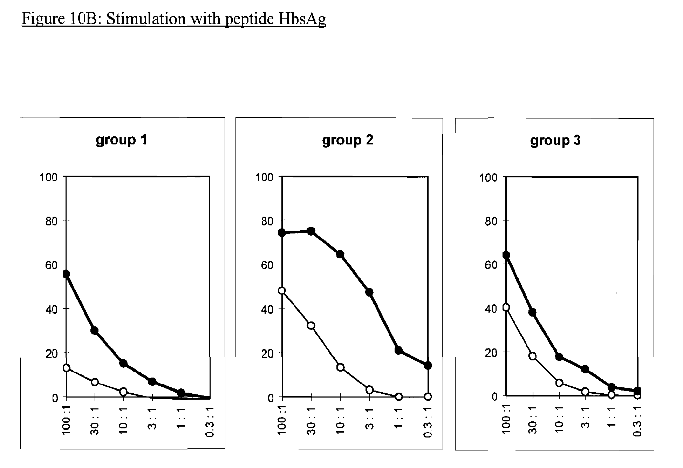

CTL responses were evaluated by using both, an HBsAg and a gp 120 CTL epitope peptide. In both cases, CTL could be detected after immunization of group 1 with CpG alone (Figure 10). However, addition of DQS21 resulted in a considerable increase in CTL for both antigens (Figure 10). The presence of an o/w emulsion either neutralized the positive effect of DQS21 (gp120) or increased the background of the in vitro assay (HBsAg).

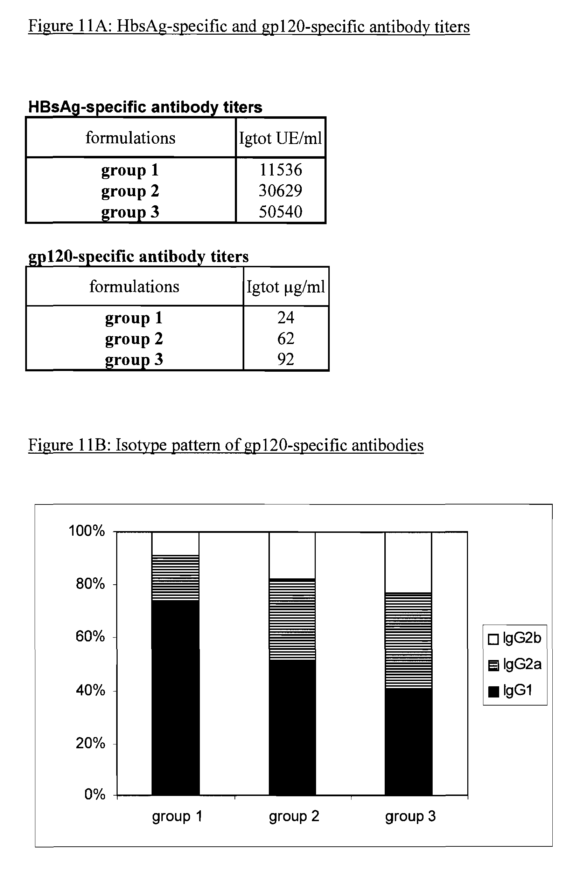

Antibody responses to HBsAg and gp 120 increased by addition of DQS21 to the CpG adjuvant (Figure 11A). A further increase was observed when an o/w emulsion was included in the formulation (Figure 11A). Addition of DQS21 to CpG shifted the gp120 isotype profiles towards a more pronounced T_{H1} bias (Figure 11B), while the impact on the HBsAg isotype profiles was less pronounced in this experiment.

### 5. Conclusions

Immunization with RTS,S and gp 120 formulated with the combination of CpG and DQS21 results in strong antigen-specific immune responses. The combination of the adjuvant components CpG and DQS21
- enhances lymphoproliferation responses
- increases CTL activity
- augments antibody titers and T_{H1} isotype patterns
as compared to the single components.

### EXAMPLE 5. Therapeutic potential of CpG and/or DQS21 formulations in TC1 tumour model

### 1. Experimental design

Four groups of 10 mice C57b1/6 received 10e6 (200 µl) TC1 cells (E7 expressing tumour cells) subcutaneous at day 0 in the flank.
Mice were then vaccinated twice at day 14 and 21 after the tumour challenge, with 5 µg of formulated PD1/3E7 HPV16 injected intra-footpad. Tumour growth was measured individually twice a week.
Groups of mice:
1. No vaccine
2. PD1/3E7 + CPG (10 µg ODN 2006)
3. PD1/3E7 + DQS21 (0.5 µg)
4. PD1/3 E7 + CPG + DQS21

The tumour growth was monitored by measuring individual tumours, twice a week.

### 2. Formulations

Formulations were performed the days of injections. The volume of injection for one mouse was 100 µl. When needed, PD1/3E7 (5 µg) was diluted with H₂O and PBS pH 7.4 for isotonicity. After 5 min., if needed QS21 (0.5 µg) mixed with liposomes in a weight ratio QS21/cholesterol of 1/5 (referred to as DQ) was added to the formulation. 30 min later, for the formulation with the oligo, 10 µg of CpG (ODN 2006) was added 30 min prior addition of 1 µg/ml of thiomersal as preservative.

| |
|---|
| H₂O + PBS pH 7.4 + PD1/3E7 - _{5 min} + DQ _{30 min} + CpG - ₃₀ₘᵢₙ - Thio |

### 3. Results

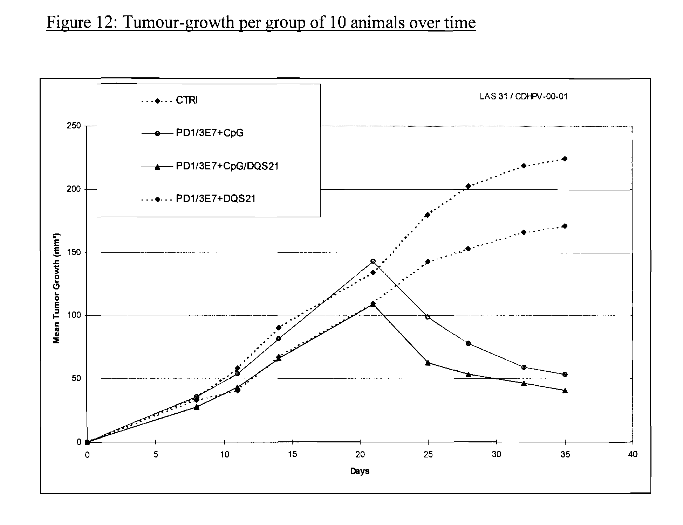

The evolution of the mean tumour growth per groups of 10 animals over time is shown in Figure 12. 100% of the animals that received a tumour challenge of 10e6 TC1 cells progressively developed growing tumour.
70-80% of the non vaccinated animals or of the animals vaccinated with the E7 protein in DQS21 died by day 35.

Two vaccinations with the E7 protein formulated in DQS21 had almost no effect on tumour growth. On the contrary, 2 vaccinations, IFP (day 14,21) with 5 µg ProtD 1/3 E7 HPV16 in CPG adjuvant induced the regression of these pre-established tumours and protect mice from dying: 70- 80% of the mice were still alive at day 35. The combination of the 2 immunostimulants CPG and DQS21 showed a slight beneficial effect over the CpG used alone.
1. An adjuvant composition comprising a saponin and an immunostimulatory oligonucleotide.
2. An adjuvant composition according to claim 1 further comprising a carrier.
3. An adjuvant composition as claimed in claim 1 or 2, wherein said saponin is selected from the group comprising Quil A, or purified saponins such as QS21, QS7, QS17; β-escin, or digitonin.
4. An adjuvant composition as claimed in any one of claims 1 to 3, wherein said immunostimulatory oligonucleotide comprises a Purine, Purine, C, G, pyrimidine, pyrimidine sequence.
5. An adjuvant composition as claimed in claims 1 to 4, wherein said immunostimulatory oligonucleotide is selected from the group comprising: TCC ATG ACG TTC CTG ACG TT (SEQ ID NO:1); TCT CCC AGC GTG CGC CAT (SEQ ID NO:2); ACC GAT GAC GTC GCC GGT GAC GGC ACC ACG (SEQ ID NO:3); TCG TCG TTT TGT CGT TTT GTC GTT (SEQ ID NO:4); TCC ATG ACG TTC CTG ATG CT (SEQ ID NO:5).
6. An adjuvant composition according to claim 1 to 4, wherein the immunostimulatory oligonucleotide contains at least two unmethylated CG repeats being separated at least by 3 nucleotides.
7. An adjuvant composition according to claim 6, wherein the immunostimulatory oligonucleotide contains at least two unmethylated CG repeats being separated by 6 nucleotides.
8. An adjuvant composition as claimed in any one of claims 2 to 7, wherein said carrier is a particulate carrier selected from the group comprising mineral salts, emulsions, polymers, liposomes, ISCOMs.
9. A vaccine composition comprising an adjuvant composition as claimed in any one of claims 1 to 8, further comprising an antigen.
10. A vaccine composition as claimed in claim 9, wherein said antigen is derived from an organism selected from the group comprising: Human Immunodeficiency Virus, Varicella Zoster virus, Herpes Simplex Virus type 1, Herpes Simplex virus type 2, Human cytomegalovirus, Dengue virus, Hepatitis A, B, C or E, Respiratory Syncytial virus, human papilloma virus, Influenza virus, Hib, Meningitis virus, Salmonella, Neisseria, Borrelia, Chlamydia, Bordetella, Streptococcus, Mycoplasma, Mycobacteria, Haemophilus, Plasmodium or Toxoplasma, stanworth decapeptide; or Tumour associated antigens (TAA), MAGE, BAGE, GAGE, MUC-1, Her-2 neu, CEA, PSA, KSA, or PRAME; or a self peptide hormone, GnRH.
11. A vaccine composition as claimed in claim 9, wherein said antigen is derived from the group comprising (a) tumour associated antigens PSMA, PSCA, tyrosinase, survivin, NY-ESO1, prostase, PS108, RAGE, LAGE, HAGE; (b) or the N terminal 39 -43 amino acid fragment (Aβ) of the amyloid precursor protein; (c) or antigens associated to atherosclerosis.
12. A vaccine composition as claimed in claims 9 to 11 wherein the vaccine is administered systemically.
13. A vaccine composition as claimed in claims 9 to 11 wherein the vaccine is administered mucosally.
14. A vaccine composition as claimed in claim 13 wherein the saponin of the adjuvant composition is haemolytic.
15. A delivery device pre-filled with the vaccine of claims 9 to 11, said device being designed to administer the vaccine systemically.
16. A method of inducing an immune response in an individual, comprising the systemic administration of a safe and effective amount of the vaccine composition as claimed in claims 9 to 11.
17. A method of treatment of an individual susceptible to or suffering from a disease by the administration to an individual of an effective amount of the vaccine as claimed in any one of claims 9 to 14.
18. A method of treatment as claimed in claim 17, wherein the administration of the vaccine is through a systemic route.
19. A method of treatment of an individual suffering from a disease selected from the group comprising prostate, breast, colorectal, lung, pancreatic, renal, ovarian or melanoma cancers; non-cancer chronic disorders, allergy, Alzheimer, atherosclerosis, comprising the administration of a vaccine as claimed in any one of claims 9 to 11.
20. A method for preventing an individual suffering from contracting a disease selected from the group comprising prostate, breast, colorectal, lung, pancreatic, renal, ovarian or melanoma cancers; non-cancer chronic disorders, allergy, Alzheimer, atherosclerosis, comprising the administration of a vaccine as claimed in any one of claims 9 to 11.
21. A method of treatment as claimed in claims 19 and 20, wherein the vaccine is administered via a systemic route.
22. A vaccine as claimed in claim 9 or 11 for use as a medicament.
23. Use of a combination of a saponin and a CpG molecule in the manufacture of a vaccine for the prophylaxis and the treatment of viral, bacterial, parasitic infections, allergy, cancer or other chronic disorders.
24. Use of combination of a saponin, an immunostimulatory oligonucleotide and a carrier in the manufacture of a vaccine for the prophylaxis and the treatment of viral, bacterial, parasitic infections, allergy, cancer or other chronic disorders.
25. A method of inducing a systemic antigen specific immune response in a mammal, comprising administering to a mucosal surface of said mammal a composition comprising an antigen and a haemolytic saponin and a CpG molecule.
26. Method of making an adjuvant composition comprising admixing a saponin with an immunostimulatory oligonucleotide.
27. Method of making an adjuvant composition comprising admixing a saponin, an immunostimulatory oligonucleotide, and a carrier.
28. Method of making a vaccine comprising admixing the following (a) a saponin, (b) an immunostimulatory oligonucleotide, and (c) an antigen.
29. Method of making a vaccine comprising admixing the following (a) a saponin, (b) an immunostimulatory oligonucleotide, (c) a carrier and (d) an antigen.

## Claims

1. A vaccine composition comprising:
RTS,S and
an adjuvant composition comprising a saponin and an immunostimulatory oligonucleotide containing an unmethylated CpG motif.

2. A vaccine according to claim 1, wherein the saponin is QS 21.

3. A vaccine according to claim 1 or 2, wherein the immunostimulatory oliogonucleotide is selected from the group comprising:
TCC ATG ACG TTC CTG ACG TT (CpG 1826)
TCT CCC AGC GTG CGC CAT (CpG 1758)
ACC GAT GAC GTC GCC GGT GAC GGC ACC ACG
TCG TCG TTT TGT CGT TTT GTC GTT (CpG 2006)
TCC ATG ACG TTC CTG ATG CT (CpG 1668)

4. A vaccine according to any one of claims 1 to 3, which further comprises 3D-MPL.

5. A vaccine according to any one of claims 1 to 4, wherein the adjuvant composition is an oil in water emulsion.

6. A vaccine according to any of claims 1 to 4, wherein the adjuvant is a liposomal formulation.

7. A vaccine composition according to any one of claims 1 to 6, wherein the immunostimulatory oliogonucleotide is in range 1-1000 µg per dose.

8. A vaccine composition according to any one of claims 1 to 7, wherein the saponin is present in the range 1-100 µg per dose.


SEQUENCE LISTING

<110> Friede, Martin
Garcon, Nathalie
Hermand Philippe

<120> Adjuvant formulation

<130> B45181

<160> 5

<170> FastSEQ for Windows Version 3.0

<210> 1
<211> 20
<212> DNA
<213> Human

<400> 1
tccatgacgt tcctgacgtt 20

<210> 2
<211> 18
<212> DNA
<213> Human

<400> 2
tctcccagcg tgcgccat 18

<210> 3
<211> 30
<212> DNA
<213> Human

<400> 3
accgatgacg tcgccggtga cggcaccacg 30

<210> 4
<211> 24
<212> DNA
<213> Human

<400> 4
tcgtcgtttt gtcgttttgt cgtt 24

<210> 5
<211> 20
<212> DNA
<213> Human

<400> 5
tccatgacgt tcctgatgct 20